Europäisches Patentamt

European Patent Office

Office européen des brevets

(19)

(11) EP 0 923 950 A2

(12) EUROPEAN PATENT APPLICATION

(43) Date of publication:
23.06.1999 Bulletin 1999/25

(51) Int Cl.⁶: **A61L 2/18**, C11D 3/48, C11D 3/33, C11D 3/37

(21) Application number: 98310417.5

(22) Date of filing: 18.12.1998

(84) Designated Contracting States:
AT BE CH CY DE DK ES FI FR GB GR IE IT LI LU MC NL PT SE
Designated Extension States:
AL LT LV MK RO SI

(30) Priority: 18.12.1997 JP 34927397
30.10.1998 JP 31017598

(71) Applicant: TOMEY CORPORATION
Nagoya-shi, Aichi-ken (JP)

(72) Inventors:
• Ibaraki, Keiko, c/o TOMEY CORP. General-Lab.
Nagoya-shi, Aichi-ken (JP)
• Mizuno, Hideto, c/o TOMEY CORP. General-Lab.
Nagoya-shi, Aichi-ken (JP)
• Goshima, Takehiko,
c/o TOMEY CORP. General-Lab.
Nagoya-shi, Aichi-ken (JP)
• Shimbo, Keiko, c/o TOMEY CORP. General-Lab.
Nagoya-shi, Aichi-ken (JP)

(74) Representative:
Paget, Hugh Charles Edward et al
MEWBURN ELLIS
York House
23 Kingsway
London WC2B 6HP (GB)

(54) **Liquid agent for contact lens**

(57) A liquid agent for a contact lens, containing at least one disinfecting component selected from the group consisting of Polyquaternium-6, Polyquaternium-7, Polyquaternium-16 and Polyquaternium-22, and a nonionic tonicity agent and/or an amino acid.

**Description**

The present application is based on Japanese Patent Applications Nos. 9-349273 and 10-310175 filed December 18, 1997 and October 30, 1998, respectively, the content of which is incorporated hereinto by reference.

BACKGROUND OF THE INVENTION

Field of the Invention

The present invention relates to a liquid agent for a contact lens, which exhibits an excellent disinfecting effect and assures a sufficiently high degree of safety to the eyes of the user, and which liquid agent can be suitably used as a cleaning and storing or preserving liquid for the contact lens.

Discussion of the Related Art

The contact lenses worn on the eyes of the user is likely to be soiled with deposits such as proteins and lipids which are included in the tear. The contact lenses need to be cleaned to remove such deposits therefrom. Further, during a long period of use of the contact lenses, microorganisms such as bacteria tend to adhere to and proliferate on the surfaces of the contact lenses while the contact lenses are stored after they have been removed from the eyes. Such microorganisms may cause infectious diseases, giving adverse influences on the eyes of the user. In view of the above, the contact lenses need to be cleaned before they are worn on the eyes. In particular, it is indispensable to disinfect soft contact lenses since the microorganisms are likely to proliferate on the surfaces of the soft contact lenses more often than those of the hard contact lenses, increasing a risk of causing the infectious diseases.

For disinfecting the contact lenses, there have been practiced a thermal disinfecting method using a suitable boiling device for boiling the contact lenses, and a chemical disinfecting method using a suitable chemical disinfectant. The thermal disinfecting method requires a relatively long period of time to disinfect the contact lenses, using the boiling device. Accordingly, in recent years, the chemical disinfecting method has been attracting a greater attention than the thermal disinfecting method.

In the chemical disinfecting method, the contact lenses are immersed in a liquid agent which exhibits a chemical disinfecting effect, so that the contact lenses can be sufficiently disinfected. Such a liquid agent is required to exhibit not only a high disinfecting effect, but also low toxicity to the eyes since the contact lenses are immersed in the liquid agent for a long time for disinfection.

As the chemical disinfectant used in the chemical disinfecting method, chlorhexidine, quaternary ammonium salt and thimerosal are disclosed in JP-A-52-109953, JP-A-62-153217 and JP-A-63-59960. In order to obtain a sufficiently high disinfecting effect, these chemical disinfectants are included generally at a relatively high concentration in the liquid agent. In this case, the chemical disinfectants are likely to be uptaked in or adsorbed on the contact lenses, especially the soft contact lenses. It is reported that the eyes of the lens wearer suffer from troubles due to direct contact thereof with the contact lenses on which the disinfectants are adsorbed after the disinfecting treatment ("Journal of Japan Contact Lens Society" 34:267-276, 277-282, 1992, 35:219-225, 1993, 36:57-61, 1994, 37:35-39, 154-157, 1995).

To avoid the above problem, the contact lens may be disinfected by using a contact lens liquid agent in which the chemical disinfectant (germicide) is included at a relatively low concentration for assuring safety of the eyes. In this case, however, the disinfecting effect to be exhibited by the liquid agent is inevitably lowered, whereby the liquid agent cannot provide a satisfactory disinfecting or sterilizing effect, causing contamination of the contact lenses by the microorganisms.

In order to solve the problem of adsorption of the disinfectants on the contact lenses, it is proposed to use a high molecular weight quaternary ammonium which exhibits a high disinfecting effect. By using the quaternary ammonium having a high molecular weight, the entry of the germicide into the contact lens is prevented. Examples of such a method are disclosed in JP-B-2-54804 which teaches the use of Polyquaternium-1, and U.S. Patents Nos. 4361548 and 4443429, which teach the use of Polyquaternium-6. None of the germicides disclosed in these patents, however, exhibit a satisfactory disinfecting effect when used in the formulations and compositions taught in those patents. Therefore, it has been desired to provide a contact lens liquid agent capable of exhibiting a sufficiently high disinfecting effect and a high degree of safety to the eyes.

SUMMARY OF THE INVENTION

It is therefore an object of the present invention to provide a liquid agent for a contact lens, which is capable of providing sufficiently high disinfecting efficacy while having a low degree of toxicity to the eyes, and which can be safely used in disinfecting the contact lens.

The inventors have made an extensive study on various known cation polymers which are considered to have an antimicrobial activity or a disinfecting effect, in an attempt to utilize those cation polymers in a contact lens liquid agent as a disinfecting or sterilizing component. The study has revealed that some kinds of the cation polymers which are used as the materials of hair-care and skin-care cosmetics and which are called "Polyquaternium" under the CTFA (The Cosmetic Toiletry and Fragrance Association, Washington D.C.), have a low degree of toxicity to the eyes and a high degree of safety suitable as the disinfecting component to be used in the contact lens liquid agent. Further, the inventors have found that a combined use of a suitable cation polymer with a nonionic tonicity agent and/or an amino acid in the contact lens liquid agent effectively intensifies or enhances the antimicrobial activity or the disinfecting action of the cation polymer.

The above object of the present invention may be attained according to the principle of the invention, which provides a liquid agent for a contact lens, containing at least one disinfecting component selected from the group consisting of Polyquaternium-6, Polyquaternium-7, Polyquaternium-16 and Polyquaternium-22, and a nonionic tonicity agent and/or an amino acid.

The contact lens liquid agent according to the present invention contains suitably selected Polyquaternium as a component effective for disinfecting or sterilizing the contact lens, so that it exhibits an excellent disinfecting action and a high degree of safety owing to the considerably low toxicity of the Polyquaternium. Further, the combined use of the Polyquaternium and the nonionic tonicity agent and/or the amino acid provides a synergistically enhanced disinfecting effect, whereby the present contact lens liquid agent can exhibit an excellent disinfecting effect even when the concentration of the Polyquaternium included therein is relatively low.

Preferably, the disinfecting component is contained in the liquid agent at a concentration of 0.00000001-1.0 w/v%, and the nonionic tonicity agent and/or the amino acid is/are contained at a concentration of 0.01-20 w/v%.

In the present invention, at least one of glycerin, propylene glycol and mannitol is preferably used as the nonionic tonicity agent while a low molecular weight amino acid having a molecular weight of 75-250 is preferably used as the amino acid. This arrangement is effective to synergistically enhance the disinfecting activity of the suitably selected Polyquaternium.

The present contact lens liquid agent may further contain a buffer. Owing to the use of the buffer together with the suitable Polyquaternium (as the disinfecting component) and the nonionic tonicity agent and/or the amino acid, the disinfecting and sterilizing effect to be exhibited by the Polyquaternium is significantly enhanced. As the buffer, a borate buffer, a phosphate buffer, a citrate buffer or a tris(hydroxymethyl)aminomethane buffer is preferably used.

DETAILED DESCRIPTION OF THE PREFERRED EMBODIMENT

The disinfecting component of the present contact lens liquid agent is selected from substances called "Polyquaternium" under the CTFA indicated above. In other words, at least one of Polyquaternium-6, Polyquaternium-7, Polyquaternium-16 and Polyquaternium-22 is used as the disinfecting component. Owing to the combined use of the suitably selected Polyquaternium and a nonionic tonicity agent and/or an amino acid, the present contact lens liquid agent exhibits a synergistically enhanced disinfecting effect.

The above-described Polyquaternium is explained in detail on pages 462-463 of "C.T.F.A International Cosmetic Ingredient Dictionary". For instance, the Polyquaternium-6 is a homopolymer of dimethyl diallyl ammonium chloride. The Polyquaternium-7 is a polymeric quaternary ammonium salt formed of acrylamide and dimethyl diallyl ammonium chloride. The Polyquaternium-16 is a polymeric quaternary ammonium salt formed of methylvinylimidazolium chloride and vinylpyrrolidone. The Polyquaternium-22 is a copolymer of dimethyl diallyl ammonium chloride and acrylic acid.

Among various kinds of the Polyquaternium indicated above, the Polyquaternium-6 which is the homopolymer of dimethyl diallyl ammonium chloride, and the Polyquaternium-7 which is a copolymer of dimethyl diallyl ammonium chloride are preferably used in the present contact lens liquid agent since they exhibit a high disinfecting effect and assure a high degree of safety. As the Polyquaternium-6, "Merquat-100" which is available from CALGON CORPORATION, U.S.A., and which is used as a material of hair-care and skin-care products is preferably used.

The Polyquaternium suitably selected as the disinfecting component is used in combination with the nonionic tonicity agent and/or the amino acid, so that the antimicrobial action of the Polyquaternium is effectively enhanced, so that the contact lens liquid agent of the present invention can exhibit a sufficiently high disinfecting effect even if the concentration of the disinfecting component is considerably low, e.g., as low as 0.00000001 w/v%, as compared with that of the conventional contact lens liquid agents. In the present invention, the disinfecting component is included in the contact lens liquid agent at a concentration of generally at least 0.00000001 w/v%, preferably at least 0.0000001 w/v%, more preferably at least 0.000001 w/v%. The upper limit of the disinfecting component included in the present contact lens liquid agent is generally 1.0 w/v%, preferably 0.1 w/v%, more preferably 0.01 w/v%.

The nonionic tonicity agent used in combination with the suitable Polyquaternium as the disinfecting component is selected from among various known nonionic tonicity agents such as glycerin, propylene glycol, mannitol, polyethylene glycol (having an average molecular weight of 100-400), glucose, diethylene glycol, sorbitol, xylitol and cyclodextrin. In addition, any other known nonionic tonicity agents may be used, provided that they assure sufficiently high safety suitable for the present contact lens liquid agent. Any one of, or any combination of, those nonionic tonicity agents may be employed. In particular, at least one of glycerin, propylene glycol and mannitol is preferably used as the nonionic tonicity agent to be included in the present contact lens liquid agent. The nonionic tonicity agent is also effective to adjust the osmotic pressure of the contact lens liquid agent. The osmotic pressure of the present contact lens liquid agent is adjusted to within a range of 200-400 mOsm, which substantially corresponds to a physiological osmotic pressure, to thereby prevent the eye irritation which would be otherwise caused when the contact lenses which have been treated by the liquid agent are worn on the eyes.

The amino acid used in combination with the suitable Polyquaternium as the disinfecting component is selected from among various known compositions, each of which has a carboxyl group and an amino group in each molecule thereof. As the amino acid, it is preferable to use a low molecular weight amino acid having a molecular weight of 75-250 such as alanine, $\alpha$-aminoadipic acid, $\alpha$-aminobutyric acid, $\alpha$-aminoisobutyric acid, arginine, asparagine, aspartic acid, isoleucine, creatine, glutamine, glutamic acid, glycine, histidine, cystine, tyrosine, tryptophan, valine, methionine, lysine, leucine, ornithine, phenylalanine, proline, phosphoserine, sarcosine, threonine, cysteine, serine, and salts thereof. Particularly preferably used are sodium L-glutamate, glycine, DL-alanine, DL-serine, L-histidine and L-glutamine. In particular, the sodium L-glutamate or glycine is preferably used as the low molecular weight amino acid. The amino acid also functions as a buffer, and it does not give an adverse influence on the size of the contact lens which is immersed in the liquid agent, so that the amino acid can be preferably used in the present contact lens liquid agent.

The nonionic tonicity agent and/or the amino acid used in combination with the suitable Polyquaternium is/ are included in the present contact lens liquid agent generally at a concentration of 0.01-20 w/v%, preferably at a concentration of 0.05-10 w/v%. If the amount of the nonionic tonicity agent and/or the amino acid is too small, the osmotic pressure of the contact lens liquid agent is undesirably low. On the other hand, an excessive amount of the nonionic tonicity agent and/or the amino acid increases the osmotic pressure of the liquid agent to an undesirably high level.

The pH of the present contact lens liquid agent is adjusted to be held within a substantially neutral range, namely, in a range of 5-9, preferably in a range of 6-8, to permit the disinfecting component to exhibit a sufficiently high disinfecting and sterilizing effect while preventing the eye irritation when the contact lenses treated by the liquid agent are worn on the eyes. The pH value outside the above range of 5-9 may cause an eye irritation and other troubles.

For effectively keeping the pH of the present contact lens liquid agent in the above range of 5-9 which assures the safety of the eyes, at least one buffer is added to the liquid agent. The buffer is added to the present contact lens liquid agent in order to permit the disinfecting component to exhibit a satisfactory disinfecting and sterilizing effect while avoiding the eye irritation when the contact lenses which have been treated by the contact lens liquid agent are worn on the eyes.

The buffer to be added to the present contact lens liquid agent may be suitably selected from among any known buffers such as a borate buffer, a phosphate buffer, a tris(hydroxymethyl)aminomethane buffer, and a citrate buffer. The buffer is included in the contact lens liquid agent in an amount of 0.05 w/v% or more, preferably 0.1 w/v% or more, since the buffering action of the buffer tends to be insufficient if the amount of the buffer is excessively small. On the other hand, an excessive amount of the buffer may cause an eye irritation when the contact lenses treated by the contact lens liquid agent are worn on the eyes. In view of this, the buffer is added to the liquid agent in an amount of not larger than 1.2w/v%, preferably not larger than 0.8 w/v%.

The present contact lens liquid agent may further contain a surface active agent for improving a cleaning effect to remove deposits such as lipids adhering to the contact lenses, and for increasing the viscosity of the liquid agent. Any kinds of the surface active agent such as cationic, anionic, amphoteric and nonionic may be employed at concentrations which do not adversely influence the effect of the present invention, as long as the selected surface active agent is safe to the living body without adversely influencing the material of the contact lenses.

The present contact lens liquid agent may further contain other known additives such as a chelating agent, a thickener and a protein removing agent, provided that they are safe to the living body and do not give an adverse influence on the material of the contact lenses. The additives may be employed in combination, as needed, in amounts that do not inhibit the effect of the present invention.

The present contact lens liquid agent which contains the suitably selected Polyquaternium (as the disinfecting component), the nonionic tonicity agent and/or the amino acid is/are easily prepared in a way as usually used for preparing an aqueous solution, without requiring any special procedure. Namely, the present contact lens liquid agent is obtained by dissolving each component in a predetermined amount of purified sterile water. The obtained contact lens liquid agent is transparent or clear, and is subjected to sterile filtration as needed.

The contact lens is treated with the thus obtained liquid agent in the following manner. The contact lens which has been removed from the eye is accommodated in a container filled with the present contact lens liquid agent. The contact lens is immersed for disinfection in the contact lens liquid agent for a time period of from 10 minutes to 24 hours, preferably from 30 minutes to 8 hours, more preferably from 1 hour to 6 hours. It is to be understood that the contact lens may be otherwise treated using the present contact lens liquid agent.

According to the procedure described above, the contact lens can be easily and effectively disinfected without using the conventionally required boiling device. Since the suitably selected Polyquaternium as the disinfecting component assures a high degree of safety to the eyes, the contact lens can be disinfected with safety without causing any troubles with the eyes even when the contact lens is immersed in the present contact lens liquid agent for a relatively long period of time.

The contact lens liquid agent according to the present invention can be applied to any known kinds of contact lenses such as low-water-content and high-water-content soft contact lenses, and hard contact lenses, irrespective of the materials of those contact lenses.

To further clarify the concept of the present invention, some examples of the invention will be described. It is to be understood that the invention is not limited to the details of the illustrated examples, but may be embodied with various changes, modifications and improvements, which may occur to those skilled in the art without departing from the scope of the invention defined in the attached claims. The percentage in the following examples are on a weight/volume (w/v) basis unless otherwise specified.

--Test for checking the disinfecting efficacy--

As the test bacteria or fungi, *S. m. (Serratia marcescens* ATCC 13880) or *C. a. (Candida albicans* ATCC 10231) was used. The *S. m.* was cultured by using a soybean-casein medium at 30°C for 24 hours while the *C. a.* was cultured by using a Glucose-Peptone medium at 30°C for 24 hours. Each of the thus cultured *S. m.* and *C. a.* was suspended with saline, to thereby provide a bacteria or fungi suspension of $10^8$ cfu/ml.

To evaluate the disinfecting effect of the present contact lens liquid agent, 10 ml of the liquid agent was poured into a disinfected test tube. 0.05 ml of the bacteria or fungi suspension prepared as described above was introduced into the test tube, and the test tube was stored in a thermostat kept at 23°C for a suitable period of time. Thereafter, a predetermined amount of the mixture was taken out of the test tube, and diluted with sterilized saline. For 1 ml of the diluted solution, a viable cell count was measured by a mixing dilution method. In the mixing dilution method, the mixture including the bacteria (*S.m.*) was cultured by using the soybean-casein medium at 30°C for 3 days while the mixture including the fungi *(C.a.)* was cultured by using the Glucose-Peptone medium at 30°C for 5 days. On the basis of the measured viable cell count, there was calculated a viable cell count of the mixture of the contact lens liquid agent and the bacteria or fungi suspension a predetermined time after mixing of the liquid agent and the bacteria or fungi suspension. Then, an amount of reduction of the bacteria or fungi was calculated in logarithm according to the following equation.

Reduction amount [in logarithm] =

LOG (the viable cell count per 1 ml of each bacteria or

fungi suspension immediately after the preparation) -

LOG (the viable cell count per 1 ml of each bacteria

or fungi suspension after the treatment by the contact

lens liquid agent)

<Example 1>

To examine the disinfecting efficacy of the present contact lens liquid agent, the following experiment was conducted. Initially, various specimens (Nos. 1-10) of the contact lens liquid agent were prepared so as to have respective compositions as indicated in the following Tables 1 and 2, by dissolving respective components in purified sterile water. As the disinfecting component, "Merquat 550" available from CALGON CORPORATION, U.S.A., "Luviquat FC 370" available from BASF, Germany, "SWANOL AM-101" available from Nikko Chemicals, Co., Ltd, Japan, "Merquat 280" and "Merquat 100" both available from CALGON CORPORATION, U.S.A. were used. Described in detail, the Merquat 550 is Polyquaternium-7 which is a copolymer of acrylamide and dimethyl diallyl ammonium chloride.

The Lubiquat FC 370 is Polyquaternium-16 which is a copolymer of methylvinylimidazolium chloride and vinylpyrrolidone. The SWANOL AM-101 is 2-alkyl-N-carboxymethyl-N-hydroxyethyl imidazoliumbetaine. The Merquat 280 is Polyquaternium-22 which is a copolymer of dimethyl diallyl ammonium chloride and acrylic acid. The Merquat 100 is Polyquaternium-6 which is a homopolymer of dimethyl diallyl ammonium chloride.

Each of the thus prepared specimens of the contact lens liquid agent was examined of its disinfecting efficacy against the *C.a.* Namely, the fungi reduction amount four hours after mixing with the fungi suspension was obtained in accordance with the above-described disinfecting efficacy test. The results are also indicated in the Tables 1 and 2.

## T A B L E   1

| | | Specimen Nos. | | | | |
|---|---|---|---|---|---|---|
| | | 1 | 2 | 3 | 4 | 5 |
| Composition | boric acid (%) | 0.5 | 0.5 | 0.5 | 0.5 | 0.5 |
| | borax (%) | 0.11 | 0.11 | 0.11 | 0.11 | 0.11 |
| | sodium chloride (%) | 0.115 | 0.63 | 0.115 | 0.63 | 0.115 |
| | glycerin (%) | 1.7 | ---- | 1.7 | ---- | 1.7 |
| | Merquat 550 (%) | 0.1 | 0.1 | ---- | ---- | ---- |
| | Luviquat FC 370 (%) | ---- | ---- | 0.01 | 0.01 | ---- |
| | SWANOL AM-101 (%) | ---- | ---- | ---- | ---- | 0.1 |
| fungi reduction amount (LOG cfu/ml) | | 4.5 | 0.1 | 1.2 | 0.2 | 0.3 |

T A B L E  2

| | | Specimen Nos. | | | | |
|---|---|---|---|---|---|---|
| | | 6 | 7 | 8 | 9 | 10 |
| Composition | boric acid (%) | 0.5 | 0.5 | 0.5 | 0.5 | 0.5 |
| | borax (%) | 0.11 | 0.11 | 0.11 | 0.11 | 0.11 |
| | sodium chloride (%) | 0.63 | ---- | 0.63 | 0.11 | 0.63 |
| | glycerin (%) | ---- | 2.0 | ---- | 1.7 | ---- |
| | Merquat 280 (%) | ---- | 0.001 | 0.001 | ---- | ---- |
| | Merquat 100 (%) | ---- | ---- | ---- | 0.001 | 0.001 |
| | SWANOL AM-101 (%) | 0.1 | ---- | ---- | ---- | ---- |
| fungi reduction amount (LOG cfu/ml) | | 0.3 | 3.6 | 0.1 | 4.9 | 0.1 |

As is apparent from the results of the above Tables 1 and 2, the specimens Nos. 1, 3, 7 and 9 of the contact lens liquid agent according to the present invention wherein the glycerin was added as the nonionic tonicity agent exhibited a significantly higher disinfecting effect than the specimens Nos. 2, 4, 8 and 10 of the contact lens liquid agent which did not include the glycerin. The disinfecting effect of the liquid agent specimens No. 5 and 6 which used the betaine substance as the disinfecting component was not improved even if the glycerin was added as the nonionic tonicity agent. It was thus confirmed that the betaine substance as the disinfecting component did not synergistically enhance the disinfecting effect even with the combined use with the nonionic tonicity agent (i.e., glycerin).

<Example 2>

To examine the influence of a buffer on the present contact lens liquid agent, three kinds of the liquid agent specimens Nos. 11-13 in the form of an aqueous solution were prepared, so as to have the respective compositions as indicated below.

| specimen No. 11 (pH = 7.2): | |
|---|---|
| Merquat 100 | 0.001 % |
| boric acid | 0.5 % |
| borax | 0.11 % |
| glycerin | 2.0 % |

| specimen No. 12 (pH = 7.3) | |
|---|---|
| Merquat 100 | 0.001 % |
| sodium dihydrogenphosphate (dihydrate) | 0.08 % |
| disodium hydrogenphosphate (dodecahydrate) | 0.52 % |
| glycerin | 2.0 % |

| specimen No. 13 (pH = 7.3) | |
|---|---|
| Merquat 100 | 0.001 % |
| tris(hydroxymethyl)aminomethane | 0.3 % |
| glycerin | 2.0 % |
| HCl | suitable amount |

These three specimens Nos. 11-13 of the contact lens liquid agent were examined of their disinfecting effects against the *S.m.* and *C.a.,* respectively. Namely, the bacteria and fungi reduction amounts two hours after mixing with the bacteria and the fungi suspensions were obtained. The results are indicated in the following Table 3.

TABLE 3

| Specimen Nos. | 11 | 12 | 13 |
|---|---|---|---|
| reduction amount of *S.m.* (Log cfu/ml) | 4.1 | > 4.9 | > 4.9 |
| reduction amount of *C.a.* (Log cfu/ml) | > 4.9 | 2.6 | 3.5 |

It will be understood from the above results that all of the borate buffer (included in the liquid agent specimen No. 11), the phosphate buffer (included in the liquid specimen No. 12) and the tris buffer (included in the liquid specimen No. 13) synergistically enhanced the disinfecting effect exhibited by the disinfecting component (i.e., Merquat 100).

<Example 3>

Specimens Nos. 14-19 of the contact lens liquid agent were obtained each as an aqueous solution, by using Polyquaternium-6 (Merquat 100) as the disinfecting component, and various kinds of the nonionic tonicity agents as indicated in the following Table 4. Each liquid agent specimen was examined of its disinfecting effects against the S. m. and *C.a.,* respectively, in accordance with the above-described disinfecting efficacy test. Namely, the bacteria and fungi reduction amounts two hours after mixing with the bacteria and the fungi suspensions were obtained. The results are also shown in the Table 4.

T A B L E 4

| | | Specimen Nos. | | | | | |
|---|---|---|---|---|---|---|---|
| | | 14 | 15 | 16 | 17 | 18 | 19 |
| Composition | boric acid (%) | 0.5 | 0.5 | ---- | 0.5 | 0.5 | ---- |
| | borax (%) | 0.11 | 0.11 | ---- | 0.11 | 0.11 | ---- |
| | $NaH_2PO_4 \cdot 2H_2O$ (%) | ---- | ---- | 0.08 | ---- | ---- | 0.08 |
| | $Na_2HPO_4 \cdot 12H_2O$ (%) | ---- | ---- | 0.52 | ---- | ---- | 0.52 |
| | glycerin (%) | 2.0 | ---- | ---- | 1.0 | ---- | ---- |
| | propylene glycol (%) | ---- | 1.42 | ---- | 0.71 | ---- | ---- |
| | mannitol (%) | ---- | ---- | 4.5 | ---- | ---- | ---- |
| | Merquat 100 (%) | 0.001 | 0.001 | 0.001 | 0.001 | 0.001 | 0.001 |
| bacteria or fungi reduction amounts (Log cfu/ml) | *S.m.* | 4.1 | 6.0 | 3.5 | 4.0 | 0.2 | 0.1 |
| | *C.a.* | >4.9 | 5.7 | 2.2 | >5.6 | 0.4 | 0.2 |

As is apparent from the results of the above Table 4, the specimens Nos. 14-17 of the contact lens liquid agent according to the present invention, which included various nonionic tonicity agents, exhibited a sufficiently high disinfecting effect. Namely, the addition of the nonionic tonicity agents enhanced the disinfecting effect to be exhibited by the Polyquaternium-6 (Merquat 100).

<Example 4>

Specimens Nos. 20-32 of the contact lens liquid agent according to the present invention, and specimens Nos. 33-38 of the contact lens liquid agent as comparative examples, were obtained each as an aqueous solution, by using the Polyquaternium-6 (Merquat 100) as the disinfecting component, various amino acids and various buffers, in various combinations as indicated in the following Tables 5-8. For each of the specimens, the bacteria *(S.m.)* and the fungi *(C.a.)* reduction amounts four hours after mixing with the bacteria and the fungi suspensions were obtained in accordance with the disinfecting efficacy test described above. The results are also indicated in the Tables 5-8.

T A B L E  5

| | Specimen Nos. | Present invention | | | | |
|---|---|---|---|---|---|---|
| | | 20 | 21 | 22 | 23 | 24 |
| Composition | $NaH_2PO_4 \cdot 2H_2O$ (%) | 0.08 | ---- | ---- | ---- | 0.08 |
| | $Na_2HPO_4 \cdot 12H_2O$ (%) | 0.52 | ---- | ---- | ---- | 0.52 |
| | tris(hydroxymethyl) aminomethane (%) | ---- | 0.3 | ---- | ---- | ---- |
| | boric acid (%) | ---- | ---- | 0.27 | 0.27 | ---- |
| | borax (%) | ---- | ---- | 0.035 | 0.035 | ---- |
| | glycine (%) | 1.5 | 1.5 | 1.5 | 1.5 | 0.1 |
| | sodium chloride (%) | ---- | ---- | ---- | 0.05 | ---- |
| | nonionic surface active agent* (%) | ---- | ---- | ---- | 0.5 | ---- |
| | Merquat 100 (%) | 0.001 | 0.001 | 0.001 | 0.001 | 0.001 |
| bacteria or fungi reduction amounts (Log cfu/ml) | *S.m.* | 3.5 | 3.3 | >4.8 | 4.8 | 3.2 |
| | *C.a.* | 2.3 | >4.8 | >4.8 | >4.7 | 2.7 |

*:  polyoxyethylene-polyoxypropylene block copolymer

T A B L E  6

| | Specimen  Nos. | Present    invention | | | | |
| | | 25 | 26 | 27 | 28 | 29 |
|---|---|---|---|---|---|---|
| Composition | NaH$_2$PO$_4$·2H$_2$O (%) | ---- | ---- | 0.08 | 0.08 | 0.08 |
| | Na$_2$HPO$_4$·12H$_2$O (%) | ---- | ---- | 0.52 | 0.52 | 0.52 |
| | tris(hydroxymethyl) aminomethane (%) | ---- | 0.3 | ---- | ---- | ---- |
| | glycine (%) | 1.5 | ---- | ---- | ---- | ---- |
| | sodium L-glutamate (%) | ---- | 0.7 | ---- | ---- | ---- |
| | DL-alanine (%) | ---- | ---- | 0.78 | ---- | ---- |
| | L-glutamine (%) (%) | ---- | ---- | ---- | 1.29 | ---- |
| | methionine (%) | ---- | ---- | ---- | ---- | 1.29 |
| | propylene glycol (%) | ---- | 0.9 | 1.0 | 1.0 | 1.0 |
| | EDTA·2Na (%) | ---- | 0.05 | 0.05 | 0.05 | 0.05 |
| | nonionic surface active agent* (%) | ---- | 0.5 | 0.5 | 0.5 | 0.5 |
| | Merquat 100 (%) | 0.001 | 0.001 | 0.001 | 0.001 | 0.001 |
| bacteria or fungi reduction amounts (Log cfu/ml) | *S.m.* | 2.2 | 2.7 | 3.9 | 2.7 | 3.2 |
| | *C.a.* | >4.7 | 0.7 | 1.2 | 1.2 | 1.0 |

*:  polyoxyethylene-polyoxypropylene block copolymer

T A B L E 7

| | | Present invention | | | | |
|---|---|---|---|---|---|---|
| Specimen Nos. | | 30 | 31 | 32 | 33 | 34 |
| Composition | NaH$_2$PO$_4$·2H$_2$O (%) | 0.08 | 0.08 | ---- | 0.08 | ---- |
| | Na$_2$HPO$_4$·12H$_2$O (%) | 0.52 | 0.52 | ---- | 0.52 | ---- |
| | tris(hydroxymethyl) aminomethane (%) | ---- | ---- | ---- | 0.3 | ---- |
| | glycine (%) | ---- | ---- | 0.9 | 0.5 | ---- |
| | sodium L-glutamate (%) | ---- | ---- | ---- | 0.7 | ---- |
| | DL-serine (%) | 0.94 | ---- | ---- | ---- | ---- |
| | L-histidine (%) (%) | ---- | 1.34 | ---- | ---- | ---- |
| | propylene glycol (%) | 1.0 | 1.0 | ---- | ---- | ---- |
| | sodium chloride (%) | ---- | 0.05 | ---- | ---- | 0.9 |
| | EDTA·2Na (%) | 0.05 | 0.05 | ---- | 0.05 | ---- |
| | nonionic surface active agent* (%) | 0.5 | 0.5 | ---- | 0.5 | ---- |
| | Merquat 100 (%) | 0.001 | 0.001 | 0.02 | ---- | 0.02 |
| bacteria or fungi reduction amounts (Log cfu/ml) | *S.m.* | 3.2 | 4.6 | 3.0 | 0 | 0.1 |
| | *C.a.* | 0.8 | 1.2 | >5.7 | 0 | 0.1 |

*:  polyoxyethylene-polyoxypropylene block copolymer

## T A B L E   8

| | | Comparative examples | | | |
|---|---|---|---|---|---|
| Specimen Nos. | | 35 | 36 | 37 | 38 |
| Composition | boric acid (%) | ---- | 0.27 | ---- | ---- |
| | borax (%) | ---- | 0.035 | ---- | ---- |
| | tris(hydroxymethyl) aminomethane (%) | ---- | ---- | 0.5 | 0.3 |
| | glycine (%) | ---- | 1.5 | 0.9 | ---- |
| | sodium L-glutamate (%) | ---- | ---- | ---- | 0.7 |
| | propylene glycol (%) | ---- | ---- | ---- | 0.9 |
| | sodium chloride (%) | 0.9 | ---- | ---- | ---- |
| | EDTA·2Na (%) | ---- | ---- | ---- | 0.05 |
| | nonionic surface active agent*1 (%) | ---- | ---- | ---- | 0.5 |
| | Merquat 100 (%) | 0.001 | ---- | ---- | ---- |
| bacteria or fungi reduction amounts (Log cfu/ml) | *S.m.* | *2 | 0.1 | 0 | 0 |
| | *C. a.* | *2 | 0.2 | 0.1 | 0.1 |

*1:   polyoxyethylene-polyoxypropylene block copolymer
*2:   The amounts of the *S. m.* and the *C.a.* were increased.

It will be understood from the above results that the specimens Nos. 20-32 of the present contact lens liquid agent wherein the amino acid and/or the propylene glycol (as the nonionic tonicity agent) was/were included exhibited a sufficiently high disinfecting effect. That is, the disinfecting effect of the Polyquaternium-6 (Merquat 100) was enhanced by the combined use with the amino acid and/or the nonionic tonicity agent. In contrast, the specimens Nos. 33 and 36-38 which did not contain the Polyquaternium-6 (Merquat 100) had no disinfecting effect.

<Example 5>

The disinfecting effect was compared between the present contact lens liquid agent and commercially available soft contact lens treating liquids, A, B and C. The specimen of the present contact lens liquid agent used in this Example 5 was an aqueous solution having a pH value of 7.2 and obtained by dissolving, in purified water, 0.001% of the Polyquaternium-6 (Merquat 100), 0.5% of boric acid, 0.11% of borate and 1.7% of glycerin.
As the test bacteria and *fungi, P. a. (Pseudomonas aeruginosa* ATCC 9027), *St. a. (Staphylococcus aureus* ATCC 6538P), *S.m. (Serratia marcescens* ATCC 13880), *C. a. (Candida albicans* ATCC 10231) and *F.s. (Fusarium solani* ATCC 36031) were used. The *P. a.* and the *St. a.* were cultured by using a soybean-casein medium at 35°C for 24 hours. The *S.m.* was cultured by using the soybean-casein medium at 30°C for 24 hours. Each of the thus cultured bacteria was suspended with saline, to thereby provide a bacteria suspension of $10^8$ cfu/ml. The *C.a.* was cultured by using a Glucose-Peptone medium at 30°C for 24 hours, and suspended with saline, to thereby provide a fungi suspension of $10^8$ cfu/ml. The *F. s.* was cultured by using the Glucose-Peptone medium at 23°C for 10 days. After the cultured *F. s.* was harvested from the surface of the medium by using saline containing 0.05% of Polysorbate 80, it was filtered through sterilized polypropylene wool so as to remove hyphal fragments therefrom, thereby providing a spore suspension of $10^8$ cfu/ml.

The commercially available soft contact lens treating liquids A-C were examined of their disinfecting effects against the above-described five kinds of the bacteria and the fungi, respectively. Namely, the bacteria and the fungi reduction amounts were respectively obtained four hours after mixing with the bacteria and the fungi suspensions, and the spore suspension, in accordance with the above-described disinfecting efficacy test. Similarly, the specimen of the present contact lens liquid agent prepared as described above was examined of its disinfecting effects against the five kinds of the bacteria and the fungi, respectively. Namely, the bacteria and the fungi reduction amounts one hour, two hours and four hours after mixing with the suspensions were respectively obtained. The results are indicated in the following Tables 9 and 10.

## T A B L E  9

| | A | B | C |
|---|---|---|---|
| disinfecting component (concentration)) | Polyquaternium-1 (0.0011 %) | polyhexamethylene-biguanide (0.00005 %) | polyhexamethylene-biguanide (0.0001 %) |
| *P. a.* reduction amount (Log cfu/ml) | 1.8 | 4.5 | >4.7 |
| *St. a.* reduction amount (Log cfu/ml) | 4.7 | >3.8 | 4.8 |
| *S. m.* reduction amount (Log cfu/ml) | 1.5 | 1.8 | 1.4 |
| *C. a.* reduction amount (Log cfu/ml) | <1 | <1 | <1 |
| *F. s.* reduction amount (Log cfu/ml) | 3.2 | 3.1 | 3.6 |

T A B L E   10

| | Present invention | | |
|---|---|---|---|
| | after one hour | after two hours | after four hours |
| *P. a.* reduction amount (Log cfu/ml) | >5.4 | >5.4 | >5.4 |
| *St. a.* reduction amount (Log cfu/ml) | >4.4 | >5.4 | >5.4 |
| *S. m.* reduction amount (Log cfu/ml) | 3.7 | 4.1 | >4.1 |
| *C. a.* reduction amount (Log cfu/ml) | 4.8 | >4.9 | >4.9 |
| *F. s.* reduction amount (Log cfu/ml) | 3.8 | >4.2 | >5.1 |

It will be apparent from the results of the Tables 9 and 10 that the present contact lens liquid agent exhibited a higher disinfecting effect especially against the fungi (*C. a.* and *F. s.*), and the bacteria, *S. m.*, than the commercially available products A-C. Further, it was confirmed that the present contact lens liquid agent exhibited a satisfactory disinfecting effect within one hour.

<Example 6>

There was prepared a specimen of the present contact lens liquid agent which included the Polyquaternium-6 (Merquat 100) and the amino acid. The obtained specimen was examined of its disinfecting effects against the *P. a.*, *St. a.*, and *F. s.*, respectively, in a manner similar to that of the above Example 5. Namely, the reduction amounts of the bacteria and the fungi in the liquid agent specimen were obtained four hours after mixing with the test bacteria suspensions and the spore suspension, respectively.

The specimen of the contact lens liquid agent used in this Example 6 was obtained by dissolving, in purified water, 0.08% of $NaH_2PO_4 \cdot 2H_2O$, 0.52% of $Na_2HPO_4 \cdot 12H_2O$, 0.3% of tris(hydroxymethyl)aminomethane, 0.5% of glycine, 0.7% of sodium L-glutamate, 0.05% of EDTA·2Na, 0.5% of nonionic surface active agent (polyoxyethylene-polyoxypropylene block copolymer), and 0.001% of Merquat 100.

The results are shown in the following Table 11. As is apparent from the results, the specimen of the contact lens liquid agent according to the present invention exhibited excellent disinfecting effects against all of the test bacteria and the test fungi.

T A B L E 11

| *P. a.* reduction amount (Log cfu/ml) | *St. a.* reduction amount (Log cfu/ml) | *F. s.* reduction amount (Log cfu/ml) |
|---|---|---|
| >5.6 | >5.6 | >5.7 |

<Example 7>

The disinfecting effect of the contact lens liquid agent was examined when the concentration of the Polyquaternium-6 as the disinfecting component was relatively low. Namely, the contact lens liquid agents having different concentration values of the Polyquaternium-6 were examined of their disinfecting effects, by using the *St. a.* suspension. Namely, the reduction amount of the *St. a.* in each liquid agent was calculated two hours after mixing with the *St. a.* suspension.

The contact lens liquid agents used in this Example 7 included 0.5% of boric acid, 0.11% of borax, 1.7% of glycerin, and the Polyquaternium-6 (Merquat 100) at different concentration values, i.e., 0.000005%, 0.00001%, 0.00005% and 0.0001%. Each liquid agent was prepared as an aqueous solution, and had a pH value of 7.2.

The results are indicated in the following Table 12. It will be apparent from the results that the contact lens liquid agent exhibited a significantly high disinfecting effect even when the concentration of the Polyquaternium-6 was as low as 0.000005 %.

## T A B L E 12

| | Concentration of the Polyquaternium-6 in the liquid agent (%) | | | |
| --- | --- | --- | --- | --- |
| | 0.000005 | 0.00001 | 0.00005 | 0.0001 |
| *St. a.* reduction amount (Log cfu/ml) | 2.07 | 2.51 | 4.46 | >4.74 |

<Example 8>

The following tests [1] and [2] were conduced by using contact lenses to evaluate, in terms of safety, the Polyquaternium-6 as a representative example of various kinds of the Polyquaternium used as the disinfecting component in the present contact lens liquid agent. [1] An experiment according to an agar over lay method to observe whether or not the Polyquaternium-6 was adsorbed on the contact lens:

Commercially available soft contact lenses ("MENICON SOFT MA" and "MENICON SOFT S" both available from Menicon Co., Ltd., Japan) were prepared. Each contact lens was immersed in 2.0 ml of an aqueous solution containing 0.4% of the Polyquaternium-6 (according to the present invention), and in 2.0 ml of an aqueous solution containing 0.1% of benzalkonium chloride (as a comparative example), at room temperature for 18 hours. Thereafter, the contact lenses were taken out of the respective aqueous solutions, and placed on fibroblasts L-929 of a mouse, so as to observe whether the cells (fibroblasts) held in contact with the soft contact lenses which had been immersed in the above aqueous solutions were adversely influenced or not by the Polyquaternium-6 and the benzalkonium chloride. This experiment to examine the cell toxicity was conducted according to the agar over lay method using a Neutral Red explained below. In this experiment, the cells were evaluated as toxic when a lysis of the cells was observed or when discoloring of the Neutral Red was observed, due to the influence of the toxic substance adsorbed on the soft contact lens. On the other hand, the cells were evaluated as nontoxic when discoloring of the Neutral Red was not observed. The Neutral Red is a kind of a coloring matter to be added to cultured cells. The live cells are dyed with the Neutral Red being introduced thereinto, whereas the dead cells are not dyed with the Neutral Red being discharged therefrom.

According to the above experiment by the agar over lay method to examine the cell toxicity, the cells held in contact with the contact lens which had been immersed in the Polyquaternium-6 solution according to the present invention were evaluated nontoxic. This means that the concentration of the Polyquaternium-6 as high as 0.4% assured safety, without the toxic substance being adsorbed on the contact lens. In contrast, the cells held in contact with the contact lens which had been immersed in the benzalkonium chloride solution were evaluated toxic, giving a safety problem in practical use.

(2) Test for measuring the inhibitory rate of proliferation of cells:

For checking the safety of the present contact lens liquid agent, the following test was conducted to measure the inhibitory rate of proliferation of the cells, by using the fibroblasts L-929 of a mouse.

Initially, the fibroblasts L-929 were diluted into a cell solution of $1 \times 10^5$ cells/ml. 100 microlitters of this cell solution was poured into each of 96 wells of a tissue culture plate, each of the wells having a flat bottom surface. The tissue culture plate was stored in a $CO_2$-incubator kept at 37°C for 4 hours for culturing the cell solution accommodated in the wells. After it was confirmed that the cells adhered to the bottom surface of each well, 10 microlitters of the following test solutions were added to the wells, and the mixtures were cultured at 37°C. After 3 days, the medium in each well was removed, and washed. According to the protein-quantitative analysis method (Lowry method), an amount of the live cells was measured as an amount of protein. The cell proliferation inhibitory rate was calculated as a ratio of the amount of the protein in each well to which the corresponding test solution was added, with respect to an amount of protein (live cells) in the well to which the test solutions were not added. The following four aqueous solutions Nos. 1-4 were used as the test solutions.

No. 1: phosphate buffer + Polyquaternium-6 (0.001%) + glycerin (1.7%)
No. 2: phosphate buffer + Polyquaternium-6 (0.001%) + propylene glycol (1.42%)
No. 3: Polyquaternium-6 (0.001%) + propylene glycol (1.42%)
No. 4: phosphate buffer (as a control)

The results of the above cell proliferation inhibitory test reveled that the inhibitory rates were 0% in all of the above test solutions. Thus, it was confirmed that the present contact lens liquid agent had a high degree of safety without inhibiting the proliferation of the cells.

## Claims

1. A liquid agent for a contact lens, containing at least one disinfecting component selected from the group consisting of Polyquaternium-6, Polyquaternium-7, Polyquaternium-16 and Polyquaternium-22, and a nonionic tonicity agent and/or an amino acid.

2. A liquid agent according to claim 1, wherein said disinfecting component is contained at a concentration of 0.00000001-1.0 w/v%.

3. A liquid agent according to claim 1 or 2, wherein said nonionic tonicity agent and/or said amino acid is/are contained in a concentration of 0.01-20 w/v%.

4. A liquid agent according to any one of claims 1-3, wherein said nonionic tonicity agent is selected from the group consisting of glycerin, propylene glycol and mannitol.

5. A liquid agent according to any one of claims 1-4, wherein said amino acid comprises a low molecular weight amino acid having a molecular weight of 75-250.

6. A liquid agent according to claim 5, wherein said low molecular weight amino acid is selected from the group consisting of alanine, α-aminoadipic acid, α-aminobutyric acid, α-aminoisobutyric acid, arginine, asparagine, aspartic acid, isoleucine, creatine, glutamine, glutamic acid, glycine, histidine, cystine, tyrosine, tryptophan, valine, methionine, lysine, leucine, ornithine, phenylalanine, proline, phosphoserine, sarcosine, threonine, cysteine, serine, and salts thereof.

7. A liquid agent according to claim 6, wherein said salt is sodium L-glutamate.

8. A liquid agent according to any one of claims 1-7, further containing a buffer.

9. A liquid agent according to claim 8, wherein said buffer is contained in an amount of 0.05-1.2 w/v%.

10. Use of a liquid agent according to any one of claims 1 to 9, for disinfecting and/or storing a contact lens.